# EUROPEAN PATENT APPLICATION

(11) **EP 4 617 664 A1**
(43) Date of publication of application: **17.09.2025**
(21) Application number: 24163439.3
(22) Date of filing: 14.03.2024
(51) Int. Cl.: G01N 33/574, C12Q 1/6886

(54) **A BIOMARKER PANEL FOR EARLY DETECTION OF CANCER**

(71) Applicant: Siemens Healthineers AG, 91301 Forchheim (DE)
(72) Inventor: Kanne, Julian, 76829 Landau (DE); Müller-Eisert, Judith, 51503 Rösrath (DE)
(74) Representative: Siemens Healthineers Patent Attorneys

(57) **Abstract**

The present invention relates to a composition for diagnosing, detecting, or monitoring a cancer disease, comprising a nucleic acid affinity ligand and/or a peptide affinity ligand for the expression product or protein of each biomarker of a group of biomarkers comprising at least Prolactin and Transferrin, and optionally further comprising a nucleic acid affinity ligand and/or a peptide affinity ligand for the expression product(s) or protein(s) of one or more of Neuron Specific Enolase (NSE), CYFRA 21-1, Carcinoembryonic Antigen (CEA), and the glycolipid marker CA19-9. The present invention further envisages a corresponding method for diagnosing, detecting or monitoring a cancer disease in a subject, in particular early forms of breast cancer.

## Description

### TECHNICAL FIELD

The present invention relates to a composition for diagnosing, detecting, or monitoring a cancer disease, comprising a nucleic acid affinity ligand and/or a peptide affinity ligand for the expression product(s) or protein(s) of a group of biomarkers comprising at least Prolactin and Transferrin, optionally further comprising a nucleic acid affinity ligand and/or a peptide affinity ligand for the expression product(s) or protein(s) of one or more of Neuron Specific Enolase (NSE), CYFRA 21-1, Carcinoembryonic Antigen (CEA), and the glycolipid marker CA19-9. The present invention further envisages a corresponding method and assay for diagnosing, detecting or monitoring a cancer disease in a subject, as well as the use of said nucleic acid affinity ligand and/or a peptide affinity ligand for detecting, diagnosing, or monitoring a cancer disease, in particular early forms of breast cancer.

### BACKGROUND

Worldwide, 1 in 8 women carry a risk to develop breast cancer and it is the second leading cause of cancer-related death in women worldwide (Arnold et al., 2022, The Breast, 66, 15-23). Strikingly, the disease prognosis can be improved if the tumor is detected at an early stage and can be removed by surgical resection or be thoroughly monitored in case a surgical intervention is not yet deemed necessary. Whereas the overall five-year survival rate for breast cancer is 90% if the tumor is discovered early in a locally restricted stage and has not spread to lymph nodes or distant organs, this value decreases to 20-30% for metastatic breast cancer (Nardin et al., 2020, Front Oncol, 10, 864; Riggio et al., 2021, British Journal of Cancer, 124, 13-26). The participation of women in annual screening programs by the use of mammography leads to an early detection of cancer in about 7% of women and reduces the likelihood to die of breast cancer by 20% (Mann et al., 2022, Eur Radiol, 32, 4036-4045).

The early detection of breast cancer is currently approached using imaging-based techniques, in particular mammography for the overall screening population, which also represents the standard of care. Nevertheless, imaging-based techniques harbor technical, financial and operational challenges including operational restrictions by country-specific breast cancer screening guidelines, low participation rates in annual mammography screening programs, false positive results and false negative results.

There is thus a clear need to improve the early detection of breast cancer in particular through minimal-invasive tests.

### SUMMARY

The present invention addresses these needs and provides in a first aspect a composition for diagnosing, detecting, or monitoring a cancer disease, comprising a nucleic acid affinity ligand and/or a peptide affinity ligand for an expression product or protein of each biomarker of a group of biomarkers, the group comprising at least Prolactin and Transferrin. The group may optionally, in certain embodiments, further comprise a nucleic acid affinity ligand and/or a peptide affinity ligand for an expression product or a protein of one or more of Neuron Specific Enolase (NSE), CYFRA 21-1, Carcinoembryonic Antigen (CEA), and the glycolipid marker CA19-9. The invention is based on the surprising finding that the mentioned markers display an altered abundance in tumor serum samples, when compared to healthy serum samples. This panel provides the advantage that it can be used as standalone panel, e.g. to triage individuals for subsequent imaging-based detection methods, that are not included in the guidelines and to facilitate analysis for female individuals that do not follow the regular imaging-based screening schedule. It further allows to complement positive and negative findings in a minimally invasive manner, especially for individuals with dense breasts.

In a preferred embodiment, said nucleic acid affinity ligand is a set of oligonucleotides specific for a Prolactin, NSE, CYFRA 21-1, CEA, or Transferrin expression product, or an oligonucleotide probe specific for a Prolactin, Neuron Specific Enolase (NSE), CYFRA 21-1, Carcinoembryonic Antigen (CEA), or Transferrin expression product, and/or said peptide affinity ligand is an antibody specific for Prolactin, Neuron Specific Enolase (NSE), CYFRA 21-1, Carcinoembryonic Antigen (CEA), Transferrin protein or the glycolipid marker CA19-9.

In a further aspect the present invention relates to a method of diagnosing, detecting or monitoring a cancer disease in a subject, comprising at least the step of determining the level of expression of Prolactin and Transferrin, and optionally also of any one or more of NSE, CYFRA 21-1, CEA, and the glycolipid marker CA19-9 in a subject's sample, preferably comprising the additional step of determining the level of expression of Prolactin and Transferrin, and optionally also of any one or more of NSE, CYFRA 21-1, CEA, and the glycolipid marker CA19-9, in a control sample, and determining the difference of the levels of expression of Prolactin and Transferrin, and optionally also of any one or more of NSE, CYFRA 21-1, CEA, and the glycolipid marker CA19-9, in the subject's sample versus the control sample.

In yet another aspect the present invention relates to a method for diagnosing, detecting or monitoring a cancer disease in a subject, comprising: (a) determining the level of expression of Prolactin and Transferrin, and optionally also of any one or more of NSE, CYFRA 21-1, CEA, and the glycolipid marker CA19-9, in a sample of the subject; (b) determining the level of expression of a reference marker in the subject's sample; or determining the level of an internal standard; (c) optionally normalizing the determined expression level of Prolactin and Transferrin, and optionally also of any one or more of NSE, CYFRA 21-1, CEA, and the glycolipid marker CA19-9, to the expression level of the reference marker or internal standard; and (d) comparing the determined expression levels of step (a) and step (b), and optionally of step (c), wherein an increased expression level of Prolactin and a decreased expression level of Transferrin, and optionally an increased expression level of any one or more of NSE, CYFRA 21-1, CEA, and the glycolipid marker CA19-9, in the sample of the subject compared to a control sample is indicative of a cancer disease.

In a further aspect the present invention relates to a method for detecting, diagnosing or monitoring a cancer disease in a subject comprising at least the steps: (a) determining the level of expression of Prolactin and Transferrin, and optionally also of any one or more of NSE, CYFRA 21-1, CEA, and the glycolipid marker CA19-9, in a sample of the subject;
(b) determining the level of expression of Prolactin and Transferrin, and optionally also of any one or more of NSE, CYFRA 21-1, CEA, and the glycolipid marker CA19-9, in a control sample; (c) determining the difference in the expression levels of Prolactin and Transferrin, and optionally also of any one or more of NSE, CYFRA 21-1, CEA, and the glycolipid marker CA19-9, as determined in steps (a) and (b); and (d) determining the absence or presence of a cancer disease based on the results obtained in step (c).

In a further embodiment of the methods as defined above, said methods comprise the additional step of determining the expression level of one or more further biomarkers.

In yet another preferred embodiment said one or more further biomarker is TNFalpha, IGF-BP3, IGF1, IL8, IL10, IL2R and/or IL1b.

In a further embodiment said sample of a subject is a body fluid sample, preferably a blood sample such as a serum or plasma sample, a urine sample, a urine sediment sample, a breath sample, a saliva sample, a semen sample, or a nipple aspiration sample.

In yet another preferred embodiment said control sample is a sample of a healthy subject.

In another preferred embodiment said reference marker is the expression product of a housekeeping gene.

In another preferred embodiment of the present invention the levels of expression are measured by an immunoassay such as an enzyme immunoassay (EIA), a radioimmunoassay (RUA), a fluoroimmunoassay (FIA), a chemiluminescence immunoassay (CLIA), a counting immunoassay (CIA), or an Enyzme-linked Immunosorbent Assay (ELISA), wherein said immunoassay is preferably performed on an analyzer instrument, such as the Atellica CI 1900 analyzer instrument.

It is further envisaged that the method according to the invention additionally comprises the step of evaluation of (i) the subject's imaging-based data, preferably derived from an MRT, medical images and/or pathology images, or mammography analysis; and/or (ii) non-imaging-based data, such as data on age, sex, race, characteristics of cancer phenotype, histologic characteristics, histologic subtype, detectable molecular changes, preferably on the level of the genome, transcriptome, metabolome, proteome, glycome or lipidome, biomarkers and/or laboratory tests, wherein the results of such evaluation(s) contribute to the diagnostic and/or monitoring conclusion with respect to the presence of a cancer disease in the subject.

In a further aspect the present invention relates to the use of a nucleic acid affinity ligand and/or a peptide affinity ligand for an expression product or protein of each biomarker of a group of biomarkers, the group comprising at least Prolactin and Transferrin, and optionally also any one or more of NSE, CYFRA 21-1, CEA, and the glycolipid marker CA19-9, for detecting, diagnosing, or monitoring a cancer disease.

In a preferred embodiment of the composition, method, or use as defined herein above the cancer disease is breast cancer. It is particularly preferred that the cancer disease is an early form of breast cancer.

### BRIEF DESCRIPTION OF THE DRAWINGS

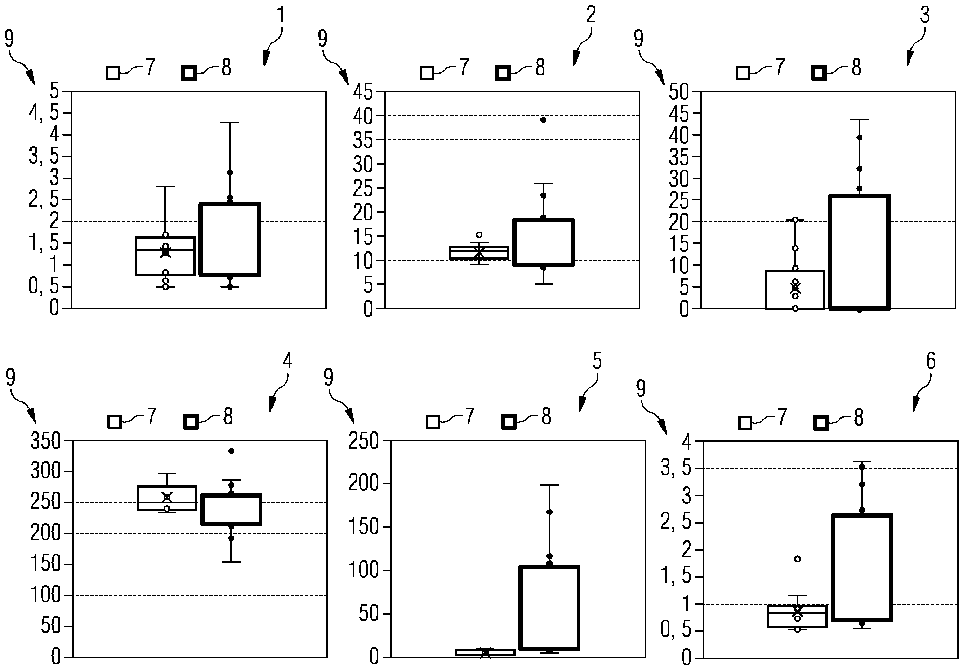

FIG. 1 shows protein expression levels of biomarker panel proteins. Protein expression levels in human serum were assessed by using commercially available immunoassays on an Atellica IM analyzer (Siemens Healthineers Diagnostics Inc.).

Boxplots display the results for the indicated proteins. Data are derived from 6 normal healthy samples and 18 to 20 tumor patient samples, respectively. Indicated is CEA **(1),** NSE **(2)** CA19-9 **(3).** Transferrin **(4),** Prolactin **(5)** and CYFRA 21-1 **(6).** The values are shown for normal/healthy **(7)** and tumor **(8)** samples. On the left hand side, the concentration in ng/ml (for CEA **(1),** NSE **(2),** Prolactin **(5)** and CYFRA 21-1 **(6)),** in mg/dL (Transferrin **(4)),** or U/ml (CA19-9 **(3))** is indicated.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i) ", "(ii)", "(iii)" or " (a) ", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a composition for diagnosing, detecting, or monitoring a cancer disease, comprising a nucleic acid affinity ligand and/or a peptide affinity ligand for an expression product or protein of each biomarker of a group of biomarkers, the group comprising at least Prolactin and Transferrin.

The term "nucleic acid affinity ligand for the Prolactin expression product" as used herein refers to one or more nucleic acid molecules being able to specifically bind to a Prolactin transcript, more preferably to a sequence of NCBI Reference number NM_000948.6 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000948.6 or a DNA sequence encoding an amino acid sequence being at least 90%, 91%, 92%, 93%, 94%, 95%, 96%, 97%, 98% or 99% identical to the sequence of UniProt Reference number P01236 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Prolactin protein" as used herein refers to a peptide molecule being able to specifically bind to the Prolactin protein. The peptide molecule may preferably be able to specifically bind to a protein or polypeptide comprising the amino acid sequence as set forth in UniProt Reference number P01236. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000948.6 or to a protein or polypeptide comprising an amino acid sequence being at least 90%, 91%, 92%, 93%, 94%, 95%, 96%, 97%, 98% or 99% identical to the sequence as set forth in UniProt Reference number P01236 or to any fragments of said sequences. Further envisaged are splice or modification variants to the above referenced sequences as known to the skilled person or derivable from suitable databases.

The term "nucleic acid affinity ligand for the Transferrin expression product" as used herein refers to one or more nucleic acid molecules being able to specifically bind to a Transferrin transcript, more preferably to a sequence of NCBI Reference number NM_001063.4 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_001063.4 or a DNA sequence encoding an amino acid sequence being at least 90%, 91%, 92%, 93%, 94%, 95%, 96%, 97%, 98% or 99% identical to the sequence of UniProt Reference number P02787 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Transferrin protein" as used herein refers to a peptide molecule being able to specifically bind to the Transferrin protein. The peptide molecule may preferably be able to specifically bind to a protein or polypeptide comprising the amino acid sequence as set forth in UniProt Reference number P02787. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_001063.4 or to a protein or polypeptide comprising an amino acid sequence being at least 90%, 91%, 92%, 93%, 94%, 95%, 96%, 97%, 98% or 99% identical to the sequence as set forth in UniProt Reference number P02787 or to any fragments of said sequences. Further envisaged are splice or modification variants to the above referenced sequences as known to the skilled person or derivable from suitable databases.

The term "expression product" as used herein refers to a transcript or an mRNA molecule generated by the expression of a gene of a biomarker according to the present invention, in particular of the Prolactin or Transferrin gene. More preferably, the term relates to a processed Prolactin or Transferrin transcript. The term "peptide" refers to any type of amino acid sequence comprising more than 2 amino acids, e.g. polypeptide structures, protein structures, protein or functional derivatives thereof. Furthermore, the peptide may, in certain embodiments, be combined with further chemical moieties or functionalities.

The term "cancer disease" as used herein refers to any cancer, tumor or under-controlled or uncontrolled cellular proliferation. It is preferred that the cancer disease is a breast cancer. Further, the cancer disease may further include the presence of pre-invasive lesions or a pre-cancerous cell population, or a predisposition for cancer or tumor. In particularly preferred embodiments the cancer is an early form of breast cancer, e.g. a breast cancer of stage 1 or 2, or pre-forms thereof.

The term "detecting a cancer disease" as used herein means that the presence of a cancer disease or disorder in an organism may be determined or that such a disease or disorder may be identified in an organism. The determination or identification of a cancer disease or disorder, e.g. breast cancer, may be accomplished by a comparison of the expression level of the biomarkers of the present invention in comparison to control levels. A cancer may be detected when the level of expression of the group of biomarkers according to the present invention comprising at least Prolactin, optionally one or more of NSE, CYFRA 21-1, CEA, and the glycolipid marker CA19-9, is increased or up-regulated, and when the level of expression of Transferrin is decreased or down-regulated in comparison to a control level as defined herein above. In a specific embodiment of the present invention a cancer may be detected if the expression level of the group of biomarkers according to the present invention comprising at least Prolactin and Transferrin is similar to an expression level of a suitable cancer reference cell.

The term "diagnosing a cancer disease" as used herein means that a subject may be considered to be suffering from a cancer disease, e.g. breast cancer, when the level of expression of at least Prolactin, optionally one or more of NSE, CYFRA 21-1, CEA, and the glycolipid marker CA19-9, is increased or up-regulated, and when the level of expression of Transferrin is decreased or down-regulated, compared to a control level, preferably if compared to a normal control level. The term "diagnosing" also refers to the conclusion reached through that comparison process.

The term "monitoring of a cancer disease" as used herein relates to the accompaniment of a diagnosed or detected cancer disease or disorder, e.g. breast cancer. The accompaniment may be performed during a treatment procedure or during a certain period of time, typically during 2 months, 3 months, 4 months, 6 months, 1 year, 2 years, 3 years, 5 years, 10 years, or any other period of time. The term "accompaniment" means that states of disease, e.g. stage 1 or 2, in particular, changes of these sates of disease may be detected by comparing the level of expression of the group of biomarkers according to the present invention comprising at least Prolactin and Transferrin in a sample to a control level, preferably a control level of expression derived from a healthy control or to the level of expression of an established, e.g. independently established, cancer cell or cell line, e.g. a breast cancer cell line, in any type of periodical time segment, e.g. every week, every 2 weeks, every month, every 2, 3, 4, 5, 6, 7, 8, 9, 10, 11 or 12 months, every 1.5 years, every 2, 3, 4, 5, 6, 7, 8,9 or 10 years, during any period of time, e.g. during 2 weeks, 3 weeks, 1, 2, 3, 4, 5, 6, 7, 8, 9, 10, 11, 12 months, 1.5, 2, 3, 4, 5, 6, 7, 8, 9, 10, 15 or 20 years, respectively. The established, e.g. independently established cancer cell, e.g. breast cancer cell, or cell line giving rise to an additional control level may be derived from samples corresponding to different stages of cancer development, e.g. stages 1 and 2 of the AJCC staging system. In a specific embodiment of the present invention the term relates to the accompaniment of a diagnosed cancer disease, e.g. breast cancer disease. The monitoring may also include the detection of the level of expression of additional genes or genetic elements, e.g. housekeeping genes such as GAPDH or PBGD etc. or comparisons with internal standards, e.g. if the analysis is performed in an automated analyzer.

In further embodiments, the composition according to the present invention additionally comprises a nucleic acid affinity ligand and/or a peptide affinity ligand for an expression product or protein of one or more of Neuron Specific Enolase (NSE), CYFRA 21-1, Carcinoembryonic Antigen (CEA), and the glycolipid marker CA19-9. For example, the composition may comprise a nucleic acid affinity ligand and/or a peptide affinity ligand for an expression product or protein of each of Transferrin and Prolactin and one, two, three or all of NSE, CYFRA 21-1, CEA, and the glycolipid marker CA19-9.

For example, in one embodiment the composition may comprise a nucleic acid affinity ligand and/or a peptide affinity ligand for an expression product) or protein of each of Prolactin and Transferrin and NSE.

In a further embodiment the composition may comprise a nucleic acid affinity ligand and/or a peptide affinity ligand for an expression product or protein of each of Prolactin and Transferrin and CYFRA 21-1.

In a further embodiment the composition may comprise a nucleic acid affinity ligand and/or a peptide affinity ligand for an expression product or protein of each of Prolactin and Transferrin and CEA.

In a further embodiment the composition may comprise a nucleic acid affinity ligand and/or a peptide affinity ligand for an expression product or protein of each of Prolactin and Transferrin and the glycolipid marker CA19-9.

In yet another group of embodiments, the composition may comprise a nucleic acid affinity ligand and/or a peptide affinity ligand for an expression product or protein of each of Prolactin, Transferrin, NSE and CYFRA 21-1, or of each of Prolactin, Transferrin, NSE and CEA, or of each of Prolactin, Transferrin, NSE and the glycolipid marker CA19-9.

In yet another group of embodiments, the composition may comprise a nucleic acid affinity ligand and/or a peptide affinity ligand for an expression product or protein of each of Prolactin, Transferrin, CYFRA 21-1 and CEA, or of each of Prolactin, Transferrin, CYFRA 21-1 and the glycolipid marker CA19-9.

In yet another group of embodiments, the composition may comprise a nucleic acid affinity ligand and/or a peptide affinity ligand for an expression product or protein of each of Prolactin, Transferrin, CEA and the glycolipid marker CA19-9.

In yet another group of embodiments, the composition may comprise a nucleic acid affinity ligand and/or a peptide affinity ligand for an expression product or protein of each of Prolactin, Transferrin, NSE, CYFRA 21-1 and CEA, or of each of Prolactin, Transferrin, NSE, CYFRA 21-1 and the glycolipid marker CA19-9, or of each of Prolactin, Transferrin, NSE, CEA and the glycolipid marker CA19-9, or of each of Prolactin, Transferrin, CEA, CYFRA 21-1 and the glycolipid marker CA19-9.

The term "nucleic acid affinity ligand for the Neuron Specific Enolase (NSE) expression product" as used herein refers to a nucleic acid molecule being able to specifically bind to a NSE transcript, more preferably to a sequence of NCBI Reference number NM_001975.3 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_001975.3 or a DNA sequence encoding an amino acid sequence being at least 90%, 91%, 92%, 93%, 94%, 95%, 96%, 97%, 98% or 99% identical to the sequence of UniProt Reference number P09104-1 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Neuron Specific Enolase (NSE) protein" as used herein refers to a peptide molecule being able to specifically bind to the NSE protein. The peptide molecule may preferably be able to specifically bind to a protein or polypeptide comprising the amino acid sequence as set forth in UniProt Reference number P09104-1. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_001975.3 or to a protein or polypeptide comprising an amino acid sequence being at least 90%, 91%, 92%, 93%, 94%, 95%, 96%, 97%, 98% or 99% identical to the sequence as set forth in UniProt Reference number P09104-1 or to any fragments of said sequences. Further envisaged are splice or modification variants to the above referenced sequences as known to the skilled person or derivable from suitable databases.

The term "nucleic acid affinity ligand for the CYFRA 21-1 expression product" as used herein refers to a nucleic acid molecule being able to specifically bind to a CYFRA 21-1 transcript, more preferably to a sequence of NCBI Reference number NM_002276.5 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_002276.5 or a DNA sequence encoding an amino acid sequence being at least 90%, 91%, 92%, 93%, 94%, 95%, 96%, 97%, 98% or 99% identical to the sequence of UniProt Reference number P08727 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the CYFRA 21-1 protein" as used herein refers to a peptide molecule being able to specifically bind to the CYFRA 21-1 protein. The peptide molecule may preferably be able to specifically bind to a protein or polypeptide comprising the amino acid sequence as set forth in UniProt Reference number P08727. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_002276.5 or to a protein or polypeptide comprising an amino acid sequence being at least 90%, 91%, 92%, 93%, 94%, 95%, 96%, 97%, 98% or 99% identical to the sequence as set forth in UniProt Reference number P08727 or to any fragments of said sequences. Further envisaged are splice or modification variants to the above referenced sequences as known to the skilled person or derivable from suitable databases.

The term "nucleic acid affinity ligand for the Carcinoembryonic Antigen (CEA) expression product" as used herein refers to a nucleic acid molecule being able to specifically bind to a CEA transcript, more preferably to a sequence of NCBI Reference number NM_002483.6 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_00248.6 or a DNA sequence encoding an amino acid sequence being at least 90%, 91%, 92%, 93%, 94%, 95%, 96%, 97%, 98% or 99% identical to the sequence of UniProt Reference number P40199 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Carcinoembryonic Antigen (CEA) protein" as used herein refers to a peptide molecule being able to specifically bind to the CEA protein. The peptide molecule may preferably be able to specifically bind to a protein or polypeptide comprising the amino acid sequence as set forth in UniProt Reference number P40199. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_002483.6 or to a protein or polypeptide comprising an amino acid sequence being at least 90%, 91%, 92%, 93%, 94%, 95%, 96%, 97%, 98% or 99% identical to the sequence as set forth in UniProt Reference number P40199 or to any fragments of said sequences. Further envisaged are splice or modification variants to the above referenced sequences as known to the skilled person or derivable from suitable databases.

The biomarker CA19-9 is a monosialoganglioside glycolipid, of the form 5-acetylneuraminyl-2-3-galactosyl-1-3-(fuco-pyranosyl-1-4)-N-acetylglucosamine. The term "peptide affinity ligand for CA19-9" as used herein refers to a peptide molecule being able to specifically bind to CA19-9. Preferably, the peptide affinity ligand for CA19-9 is an antibody specific for CA19-9. Further envisaged are antibodies which allow for a chemiluminescent immune detection.

In preferred embodiments the nucleic acid affinity ligands are or comprise a set of oligonucleotides specific for at least a Prolactin and Transferrin expression product, e.g. a Prolactin and Transferrin transcript or cDNA thereof. In further embodiments, the set of oligonucleotides may further additionally comprise entities which are specific for one or more of the expression products of NSE, CYFRA 21-1 and CEA. In additional embodiments, the nucleic acid affinity ligand is an oligonucleotide probe specific for a Prolactin or Transferrin expression product, e.g. transcript. In further embodiments, the nucleic acid affinity ligand may be a probe specific for one or more of the expression products of NSE, CYFRA 21-1 and CEA. In yet another embodiment, the peptide affinity ligand is an antibody specific for a Prolactin or a Transferrin protein. In further embodiments the peptide affinity ligand is an antibody specific for one or more of NSE, CYFRA 21-1 or CEA protein, or the glycolipid marker CA19-9.

In a further aspect the present invention relates to a method of diagnosing, detecting or monitoring a cancer disease in a subject, comprising at least the step of determining the level of expression of each of Prolactin and Transferrin, and optionally also of any one or more of NSE, CYFRA 21-1, CEA, and the glycolipid marker CA19-9 in a subject's sample.

The term "level of expression" as used herein refers to the amount of transcript produced during transcription or mRNA production and/or to the amount of protein or polypeptide generated by subsequent translation processes of a biomarker of the group of Prolactin, Transferrin, NSE, CYFRA 21-1 and CEA, as defined herein above. The present invention is thus based on the determination of the amount of nucleic acids and/or proteins corresponding to the group of biomarkers including at least Prolactin and Transferrin and one or more of NSE, CYFRA 21-1 and CEA. In certain embodiments, only the amount of nucleic acids, e.g. transcript or RNA or cDNA derived therefrom may be determined. In other embodiments, only the amount of protein may be determined. In further embodiments, both, the amount of nucleic acids and proteins may be determined. The term also refers to the amount of glycolipid marker CA19-9, which may be determined by different means, e.g. immunologically, for instance by binding to specific antibodies. In further embodiments, thus, the amount of nucleic acids and proteins and glycolipid markers may be determined, or the amount of nucleic acids and glycolipid markers or the amount of proteins and glycolipid markers may be determined.

The term "sample" as used herein refers to any suitable sample type or form. Preferably, the sample is a body fluid sample, such as a blood sample including a serum or plasma sample, or a urine sample, a urine sediment sample, a breath sample, a saliva sample, a semen sample, or a nipple aspiration sample. In further embodiments, the sample may be a tissue, e.g., tissue biopsy sample. Further envisaged are liquor, and liquid biopsy samples derived from blood/urine or other body fluids. Also envisaged is to make use of previously deposited samples. In further embodiments the sample may be a cell free DNA (cfDNA) sample. The term "cfDNA" as used herein refers to degraded DNA fragments released to body fluids such as blood plasma, urine, cerebrospinal fluid, etc., wherein the cfDNA typically has a short half-life and requires rapid processing and/or stabilization. The use of cfDNA is particularly advantageous since elevated levels of cfDNA are observed in cancer.

In certain embodiments, the sample may be a tumor sample, i.e., the proteins, glycolipids and/or nucleic acids may be extracted from a tumor of a subject. In other embodiments, the sample may be derived from a healthy subject.

The term "subject" as used herein refers to several different types of individuals such as a healthy individual that is not suspected of having a pre-invasive lesion, a pre-cancerous cell or a cancer disease; or an individual that is not suspected of having a pre-invasive lesion, a pre-cancerous cell or a cancer disease; or an individual that is suspected of having a pre-invasive lesion or a pre-cancerous cell; or an individual, that has a pre-invasive lesion or a pre-cancerous cell population but is not suspected of having a cancer disease; or an individual that has a pre-invasive lesion or a pre-cancerous cell population and is suspected of having a cancer disease; or an individual that is suspected of having a pre-invasive lesion or a pre-cancerous cell population; or an individual that has a pre-invasive lesion or a pre-cancerous cell population; or an individual that is suspected of having a cancer disease, e.g. breast cancer; or an individual that has a cancer disease. The term "patient" as used herein is meant to relate to a sub-group of the subjects as defined herein, i.e., an individual that has a pre-invasive lesion or a pre-cancerous cell population; or an individual that is afflicted by a cancer disease, e.g. breast cancer.

To "determine the level of expression" the amount of expression products as defined herein, i.e. of nucleic acids or proteins or glycolipids, is measured. Thus, the expression level(s) may be determined by a method involving the detection of an mRNA or of a transcript encoded by any of the biomarker genes, the detection of a protein encoded by any of the biomarker transcripts and/or the detection of the glycolipid of CA19-9.

For example, the nucleic acid level may be assessed by any suitable method known to the skilled person including separation of nucleic acid molecules in agarose or polyacrylamide gels, Northern blot analysis, as well as detection via DNA arrays or microarrays. Preferably, the nucleic acid level may be detected in a quantitative RT-PCR approach, preferably in a real-time PCR approach following the reverse transcription of the mRNA transcript of the biomarkers according to the present invention. Typically, as first step, a transcript is reverse transcribed into a cDNA molecule according to any suitable method known to the person skilled in the art. A quantitative or real-time PCR approach may subsequently be carried out based on a first DNA strand obtained as described above. Also envisaged are dPCR approaches.

Preferably, a nucleic acid affinity ligand according to the present invention as defined herein above may be used.

The measurement of protein levels of the biomarkers of the present invention or of any fragments, homologues or derivates derived thereof may be carried out via any suitable detection technique known in the art. Preferably, the protein level of the biomarkers of the present invention and derivatives thereof may be determined immunologically, e.g. by using an antibody specific for Prolactin, for Transferrin, and optionally by using an antibody specific for any one of NSE, CYFRA 21-1, CEA, and the glycolipid marker CA19-9. Alternatively, antibody variants or fragments may be used.

Preferably, a peptide affinity ligand according to the present invention as defined herein above may be used.

Determination of the protein levels can, for example, be accomplished by the separation of proteins from a sample on a polyacrylamide gel, followed by identification of biomarker proteins using specifically binding antibodies in a Western blot analysis. Alternatively, proteins can be separated by two-dimensional gel electrophoresis systems. Two-dimensional gel electrophoresis is well known in the art and typically involves iso-electric focusing along a first dimension followed by SDS-PAGE electrophoresis along a second dimension. The analysis of 2D SDS-PAGE gels can be performed by determining the intensity of protein spots on the gel, or can be performed using immune detection. In other embodiments, protein samples may be analyzed by mass spectroscopy. For glycolipids or glycosylated proteins staining and affinity-based methods may be used.

Within the context of the present invention specific antibodies may be immobilized on a solid phase. Proteins derived from samples to be analyzed may subsequently be mixed with the antibodies. A detection reaction may then be carried out, e.g. with a secondary antibody, preferably with a specific antibody.

Immunological tests which may be used in the context of the present invention, in particular for the diagnostic purposes of the present invention, include, for example, competitive and non-competitive assay systems using techniques such as western blots, radioimmunoassay like RIA (radio-linked immunoassay), ELISA (enzyme linked immunosorbent assay), "sandwich" immunoassays, immunoprecipitation assays, precipitin reactions, gel diffusion precipitin reactions, immunodiffusion assays, agglutination assays, e.g. latex agglutination, complement-fixation assays, immunoradiometric assays, fluorescent immunoassays, e.g. FIA (fluorescence-linked immunoassay), chemiluminescence immunoassays, electrochemiluminescence immunoassay (ECLIA) and protein A immunoassays. Such assays are routine and well known to the person skilled in the art.

Furthermore, the binding affinity of an antibody to an antigen and the off-rate of an antibody-antigen interaction may be determined by competitive binding assays. One example of a competitive binding assay is a radioimmunoassay comprising the incubation of labeled antigen (e.g., ³H or ¹²⁵I) with a suitable antibody in the presence of increasing amounts of unlabeled antigen, and the detection of the antibody bound to the labeled antigen. The affinity of the antibody of interest for a particular antigen and the binding off-rates may be determined from the data by any suitable analysis approach, e.g. by a scatchard plot analysis. Competition with a second antibody may also be determined using radioimmunoassays. In this case, the antigen may be incubated with a suitable antibody conjugated to a labeled compound (e.g., ³H or ¹²⁵I) in the presence of increasing amounts of an unlabeled second antibody.

In a preferred embodiment, the method as defined above comprises the additional step of determining the level of expression of Prolactin and Transferrin, and optionally also of any one or more of NSE, CYFRA 21-1, CEA, and the glycolipid marker CA19-9, in a control sample and determining the difference of the levels of expression of Prolactin and Transferrin, and optionally also of any one or more of NSE, CYFRA 21-1, CEA, and the glycolipid marker CA19-9, in the subject's sample versus the control sample.

The term "control sample" relates to a sample derived from a different source or subject than the test sample of the subject. The control sample may further be either a sample derived from the same tissue type or be derived from a different tissue type. Furthermore, the testing of the test sample for the expression of a reference gene and the testing of a control sample may be combined. It is preferred that the control sample is the sample of a healthy subject, i.e. a subject which is not affected by a cancer disease, e.g. by breast cancer.

The use of control samples may, in certain embodiments, yield control levels of expression of the tested biomarkers. Accordingly, the term "control level" as used herein, relates to an expression level which may be determined at the same time and/or under similar or comparable conditions as the test sample by using, for example, (a) sample(s) previously collected and stored from a subject/subjects whose condition or disease state, e.g. non-cancerous, normal or healthy is/are known, e.g. have been determined by an independent molecular, histological or physiological method known to the person skilled in the art.

By comparing the results obtained by determining the levels of expression of Prolactin and Transferrin, and optionally also of any one or more of NSE, CYFRA 21-1, CEA, and the glycolipid marker CA19-9, in a subject's sample and in a control sample, it may be determined whether a change or alteration of the level of expression is given. Depending on the nature of the control sample, it may be deduced, whether the subject is affected by a cancer disease, e.g. breast cancer. For example, in case the control sample is a sample of a healthy subject, an increased level of expression of Prolactin, and optionally also of any one or more of NSE, CYFRA 21-1, CEA, and the glycolipid marker CA19-9, and a decreased level of expression of Transferrin in the test sample in comparison to the control sample is considered to be indicative of a cancer disease, e.g. of breast cancer. In case the control sample is a sample of a cancerous subject, e.g. a breast cancer subject, a similar or identical level of expression of Prolactin and Transferrin, optionally also of any one or more of NSE, CYFRA 21-1, CEA, and the glycolipid marker CA19-9 in both samples is considered to be indicative of a cancer disease, e.g. a breast cancer disease.

The term "increased" or "increased expression level" or "up-regulated" in the context of the present invention thus denotes a rise in the expression level of a biomarker in a subject's sample compared to a reference point. Expression levels are deemed to be "increased" when the expression level in the subject's sample to be analyzed differs by, i.e. is elevated by, for example, 5%, 6%, 7%, 8%, 9%, 10%, 15%, 20%, 25%, 30%, 40%, 50%, or more than 50% in comparison to a healthy control level, or by at least 2 fold, at least 5 fold, or at least 10 fold or more in comparison to a healthy control level. If a comparison with a cancerous control level is to be carried out, an additional comparison with a healthy control level may be performed. Such an additional comparison allows for the determination of a tendency of the modification, e.g. the magnitude of an increase of the expression level may be observed and/or corresponding conclusions may be drawn.

The term "decreased" or "decreased expression level" or "down-regulated" in the context of the present invention thus denotes a decline in the expression level of a biomarker in a subject's sample compared to a reference point. Expression levels are deemed to be "decreased" when the expression level in the subject's sample to be analyzed, differs by, i.e. is reduced by, for example, 5%, 6%, 7%, 8%, 9%, 10%, 15%, 20%, 25%, 30%, 40%, 50%, or more than 50% in comparison to a healthy control level, or by 0.1 fold or 0.2 fold or at maximum 0.9 fold in comparison to a healthy control level. If a comparison with a cancerous control level is to be carried out, an additional comparison with a healthy control level may be performed. Such an additional comparison allows for the determination of a tendency of the modification, e.g. the magnitude of an decrease of the expression level may be observed and/or corresponding conclusions may be drawn.

If the expression level of more than one biomarker is determined, e.g. Prolactin and any one of Transferrin, NSE, CYFRA 21-1, CEA, and the glycolipid marker CA19-9, the obtained data may be statistically analyzed. For example, a measured increase or decrease of expression levels may be averaged or weighed according to a suitable procedure in order to generate a summary value for the increase, decrease or change. It is preferred that the contribution to said summary value are identical for all measured biomarkers.

In further preferred embodiments, determining of the level of expression of Prolactin and Transferrin, and optionally also of any one or more of NSE, CYFRA 21-1, CEA, and the glycolipid marker CA19-9, in a subject's sample is performed in an automatic analyzer. Particularly preferred is the employment of an Atellica analyzer, specifically of Atellica CI 1900 analyzer.

In a further aspect the present invention relates to a method for diagnosing, detecting or monitoring a cancer disease in a subject, comprising: (a) determining the level of expression of Prolactin and Transferrin, and optionally also of any one or more of NSE, CYFRA 21-1, CEA, and the glycolipid marker CA19-9, in a sample of the subject; (b) determining the level of expression of a reference marker in the subject's sample; or determining the level of an internal standard; (c) optionally normalizing the measured expression level of Prolactin and Transferrin, and optionally also of any one or more of NSE, CYFRA 21-1, CEA, and the glycolipid marker CA19-9, to the expression level of the reference marker or internal standard; and (d) comparing the determined expression level of step (a) and step (b), and optionally of step (c) wherein an increased expression level of Prolactin, and optionally also of any one or more of NSE, CYFRA 21-1, CEA, and the glycolipid marker CA19-9, and a decreased expression level of Transferrin in the sample of the subject compared to a control sample is indicative of a cancer disease. The determination of the level of expression may be performed as described herein above.

The term "reference marker" as used herein refers to any suitable biomarker, e.g. to a steadily expressed and continuously detectable gene, gene product, expression product, protein or protein variant in the organism of choice. The term also includes gene products such as expressed proteins, peptides, polypeptides, as well as modified variants thereof. Also encompassed are all kinds of transcripts derivable from a corresponding gene as well as modifications thereof or secondary parameters linked thereto. Alternatively or additionally, other reference parameters may also be used for reference purposes, e.g. metabolic concentrations, cell sizes etc. Typically, the reference marker is chosen as marker for which a corresponding amount of biomarkers according to the present invention may have been determined before, e.g. in cancer or non-cancer situations, or be known to the skilled person, e.g. from databases etc. Thus, there is an indirect reference to a cancerous and/or non-cancerous level of expression. In certain embodiments the reference marker is the expression product of a housekeeping gene.

The term "internal standard" as used herein refers to a protein or other type of marker, which is typically used in automatic analyzers in order to calibrate the amount of expression products, proteins or glycolipids. Similarly, also the internal standard may, in typical embodiments, be correlated to a corresponding amount of biomarkers according to the present invention which may have been determined before, e.g. in cancer or non-cancer situations, or be known to the skilled person, e.g. from databases, suitable literature sources etc.

By determining the amount of a reference marker or an internal standard, a calibration of the determined level of expression becomes possible. Accordingly, in certain embodiments, the measured expression levels of Prolactin and Transferrin, and optionally also of any one or more of NSE, CYFRA 21-1, CEA, and the glycolipid marker CA19-9, may be normalized to the expression level of the reference marker or internal standard. The normalization may be performed according to any suitable method known to the person skilled in the art, e.g. according to normalization statistical methods like the standard score, Student's T-test, studentized residual test, standardized moment text, or coefficient variation test. Typically, such tests or corresponding formula, which would be known to the person skilled in the art, would be used to standardize expression data to enable differentiation between real variations in expression levels and variations due to the measurement processes. Further envisaged are algorithmic approaches, which allow for a suitable internal comparison, being equivalent to a calibration approach.

Based on the expression results obtained in steps (a) and (b) and optionally the normalized results obtained in step (c) a comparison of the determined expression levels is performed. Upon this comparison an increase or decrease of the expression level may be detected, e.g. as defined herein above, which would be indicative of a cancer disease, e.g. a breast cancer disease.

In a further aspect the present invention relates to a method for detecting, diagnosing or monitoring a cancer disease in a subject comprising at least the steps: (a) determining the level of expression of Prolactin and Transferrin, and optionally also of any one or more of NSE, CYFRA 21-1, CEA, and the glycolipid marker CA19-9, in a sample of the subject; (b) determining the level of expression of Prolactin and Transferrin, and optionally also of any one or more of NSE, CYFRA 21-1, CEA, and the glycolipid marker CA19-9, in a control sample; (c) determining the difference in the expression levels of Prolactin and Transferrin, and optionally also of any one or more of NSE, CYFRA 21-1, CEA, and the glycolipid marker CA19-9, in steps (a) and (b); and (d) determining the absence or presence of a cancer disease based on the results obtained in step (c). The determination of the levels of expression may be performed as described herein above. The control sample may a control sample as defined herein above, preferably a sample from a healthy subject.

In preferred embodiments the steps in the method as described above are based on the use of antibodies specifically binding to Prolactin and Transferrin, and optionally also to any one or more of NSE, CYFRA 21-1, CEA, and the glycolipid marker CA19-9. Such antibodies would be known to the skilled person or can be derived from suitable literature sources.

In further specific embodiments the steps in the method as described above are specific clinical chemical testings, preferably as performed on automatic analyzers such as Atellica CI 1900.

It is preferred that the levels of expression are measured by an immunoassay such as an enzyme immunoassay (EIA), a radioimmunoassay (RUA), a fluoroimmunoassay (FIA), a chemiluminescence immunoassay (CLIA), a counting immunoassay (CIA), or an Enyzme-linked Immunosorbent Assay (ELISA). Preferably the immunoassay is performed on an analyzer instrument such as Atellica CI 1900.

In a further group of embodiments of the methods according to the invention said methods comprise the additional step of determining the expression level of one or more further biomarkers. These additional biomarkers may be any suitable biomarkers known to the skilled person, in particular biomarkers known to be relevant in the context of cancer disease, more preferably in the context of a breast cancer disease. Examples of suitable additional biomarker include TNFalpha, IGF-BP3, IGF1, IL8, IL10, IL2R and/or IL1b. These biomarkers may be detected with suitable nucleic acid affinity ligands or peptide affinity ligands as described herein above.

For example, a nucleic acid affinity ligand for the TNFalpha expression product may be used. This term refers to a nucleic acid molecule being able to specifically bind to a TNFalpha transcript, more preferably to a sequence of NCBI Reference number NM_000594.4 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000594.4 or a DNA sequence encoding an amino acid sequence being at least 90%, 91%, 92%, 93%, 94%, 95%, 96%, 97%, 98% or 99% identical to the sequence of UniProt Reference number P01375 or to a fragment of said sequence; or be capable of amplifying a corresponding expression product, e.g. as part of a set of oligonucleotides.

In a further embodiment, a peptide affinity ligand for the TNFalpha expression product may be used. This term refers to a peptide molecule being able to specifically bind to the TNFalpha protein. The peptide molecule may preferably be able to specifically bind to a protein or polypeptide comprising the amino acid sequence as set forth in UniProt Reference number P01375. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000594.4 or to a protein or polypeptide comprising an amino acid sequence being at least 90%, 91%, 92%, 93%, 94%, 95%, 96%, 97%, 98% or 99% identical to the sequence as set forth in UniProt Reference number P01375 or to any fragments of said sequences.

For example, a nucleic acid affinity ligand for the IGF-BP3 expression product may be used. This term refers to a nucleic acid molecule being able to specifically bind to a IGF-BP3 transcript, more preferably to a sequence of NCBI Reference number NM_000598.5 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000598.5 or a DNA sequence encoding an amino acid sequence being at least 90%, 91%, 92%, 93%, 94%, 95%, 96%, 97%, 98% or 99% identical to the sequence of UniProt Reference number P17936 or to a fragment of said sequence; or be capable of amplifying a corresponding expression product, e.g. as part of a set of oligonucleotides.

In a further embodiment, a peptide affinity ligand for the IGF-BP3 expression product may be used. This term refers to a peptide molecule being able to specifically bind to the IGF-BP3 protein. The peptide molecule may preferably be able to specifically bind to a protein or polypeptide comprising the amino acid sequence as set forth in UniProt Reference number P17936. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000598.5 or to a protein or polypeptide comprising an amino acid sequence being at least 90%, 91%, 92%, 93%, 94%, 95%, 96%, 97%, 98% or 99% identical to the sequence as set forth in UniProt Reference number P17936 or to any fragments of said sequences.

In a further example a nucleic acid affinity ligand for the IGF1 expression product may be used. This term refers to a nucleic acid molecule being able to specifically bind to a IGF1 transcript, more preferably to a sequence of NCBI Reference number NM_000618.5 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000618.5 or a DNA sequence encoding an amino acid sequence being at least 90%, 91%, 92%, 93%, 94%, 95%, 96%, 97%, 98% or 99% identical to the sequence of UniProt Reference number P05019 or to a fragment of said sequence; or be capable of amplifying a corresponding expression product, e.g. as part of a set of oligonucleotides.

In a further embodiment, a peptide affinity ligand for the IGF1 expression product may be used. This term refers to a peptide molecule being able to specifically bind to the IGF1 protein. The peptide molecule may preferably be able to specifically bind to a protein or polypeptide comprising the amino acid sequence as set forth in UniProt Reference number P05019. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000618.5 or to a protein or polypeptide comprising an amino acid sequence being at least 90%, 91%, 92%, 93%, 94%, 95%, 96%, 97%, 98% or 99% identical to the sequence as set forth in UniProt Reference number P05019 or to any fragments of said sequences.

For example, a nucleic acid affinity ligand for the IL8 expression product may be used. This term refers to a nucleic acid molecule being able to specifically bind to a IL8 transcript, more preferably to a sequence of NCBI Reference number NM_000584.4 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000584.4 o or a DNA sequence encoding an amino acid sequence being at least 90%, 91%, 92%, 93%, 94%, 95%, 96%, 97%, 98% or 99% identical to the sequence of UniProt Reference number P10145 or to a fragment of said sequence; or be capable of amplifying a corresponding expression product, e.g. as part of a set of oligonucleotides.

In a further embodiment, a peptide affinity ligand for the IL8 expression product may be used. This term refers to a peptide molecule being able to specifically bind to the IL8 protein. The peptide molecule may preferably be able to specifically bind to a protein or polypeptide comprising the amino acid sequence as set forth in UniProt Reference number P10145. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000584.4 or to a protein or polypeptide comprising an amino acid sequence being at least 90%, 91%, 92%, 93%, 94%, 95%, 96%, 97%, 98% or 99% identical to the sequence as set forth in UniProt Reference number P10145 or to any fragments of said sequences.

For example, a nucleic acid affinity ligand for the IL10 expression product may be used. This term refers to a nucleic acid molecule being able to specifically bind to a IL10 transcript, more preferably to a sequence of NCBI Reference number NM_000572.3 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000572.3 or a DNA sequence encoding an amino acid sequence being at least 90%, 91%, 92%, 93%, 94%, 95%, 96%, 97%, 98% or 99% identical to the sequence of UniProt Reference number P22301 or to a fragment of said sequence; or be capable of amplifying a corresponding expression product, e.g. as part of a set of oligonucleotides.

In yet another embodiment, a peptide affinity ligand for the IL10 expression product may be used. This term refers to a peptide molecule being able to specifically bind to the IL10 protein. The peptide molecule may preferably be able to specifically bind to a protein or polypeptide comprising the amino acid sequence as set forth in UniProt Reference number P22301. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000572.3 or to a protein or polypeptide comprising an amino acid sequence being at least 90%, 91%, 92%, 93%, 94%, 95%, 96%, 97%, 98% or 99% identical to the sequence as set forth in UniProt Reference number P22301 or to any fragments of said sequences.

For example, a nucleic acid affinity ligand for the IL2R expression product may be used. This term refers to a nucleic acid molecule being able to specifically bind to a IL2R transcript, more preferably to a sequence of NCBI Reference number NM_000417.3 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000417.3 or a DNA sequence encoding an amino acid sequence being at least 90%, 91%, 92%, 93%, 94%, 95%, 96%, 97%, 98% or 99% identical to the sequence of UniProt Reference number P01589 or to a fragment of said sequence; or be capable of amplifying a corresponding expression product, e.g. as part of a set of oligonucleotides.

In a further embodiment, a peptide affinity ligand for the IL2R expression product may be used. This term refers to a peptide molecule being able to specifically bind to the IL2R protein. The peptide molecule may preferably be able to specifically bind to a protein or polypeptide comprising the amino acid sequence as set forth in UniProt Reference number P01589 The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000417.3 or to a protein or polypeptide comprising an amino acid sequence being at least 90%, 91%, 92%, 93%, 94%, 95%, 96%, 97%, 98% or 99% identical to the sequence as set forth in UniProt Reference number P01589 or to any fragments of said sequences.

For example, a nucleic acid affinity ligand for the IL1b expression product may be used. This term refers to a nucleic acid molecule being able to specifically bind to a IL1b transcript, more preferably to a sequence of NCBI Reference number NM_000576.3 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000576.3 or a DNA sequence encoding an amino acid sequence being at least 90%, 91%, 92%, 93%, 94%, 95%, 96%, 97%, 98% or 99% identical to the sequence of UniProt Reference number P01584 or to a fragment of said sequence; or be capable of amplifying a corresponding expression product, e.g. as part of a set of oligonucleotides.

In a further embodiment, a peptide affinity ligand for the IL1b expression product may be used. This term refers to a peptide molecule being able to specifically bind to the IL1b protein. The peptide molecule may preferably be able to specifically bind to a protein or polypeptide comprising the amino acid sequence as set forth in UniProt Reference number P01584. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000576.3 or to a protein or polypeptide comprising an amino acid sequence being at least 90%, 91%, 92%, 93%, 94%, 95%, 96%, 97%, 98% or 99% identical to the sequence as set forth in UniProt Reference number P01584 or to any fragments of said sequences.

In a further embodiment the diagnostic and/or monitoring conclusion with respect to the presence or absence of a cancer disease in the subject may additionally be based on the evaluation of further data. Such further data may, for example, be the subject's imaging-based data. Examples of contemplated imaging-based data are mammography analysis data, MRT data, medical images and/or pathology images. These data, which may themselves already be connected to or translated into a diagnostic output for a subject may be combined with the conclusions derived from the method of diagnosing, detecting or monitoring a cancer disease as described herein above. The results of the evaluation(s) contribute to the diagnostic and/or monitoring conclusion with respect to the absence or presence of a cancer disease in the subject. For example, these data may be combined with conclusions and other data entities, e.g. via multimodal data integration. In certain embodiments, the integration may be performed as early, intermediate or late fusion. The present invention preferably contemplates a late fusion in which, for example, a cancer detection is combined with an imaging-based score, e.g., using a logistic regression. Also envisaged are early and intermediate fusion approaches. In a specific embodiment the combination of input data may, based on suitable weighing algorithms, result in an overall diagnostic or monitoring conclusion.

In the alternative or in addition, i.e., in a further preferred embodiment the diagnostic or monitoring conclusion with respect to the absence or presence of a cancer disease in the subject may additionally be founded on non-imaging-based data, i.e. such non-imaging data may suitable be evaluated. Examples of contemplated non-imaging-based data are data on age, sex, race, characteristics of cancer phenotype, histologic characteristics, histologic subtype, detectable molecular changes, preferably on the level of the genome, transcriptome, metabolome, proteome, glycome or lipidome, biomarkers and/or laboratory tests. These data may be combined with the conclusions derived from the method of diagnosing, detecting or monitoring a cancer disease as described herein above and optionally with the conclusions including the imaging-based data. The results of the evaluation(s) accordingly contribute to the diagnostic and/or monitoring conclusion with respect to the presence of a cancer disease in the subject. For example, the data may be combined with conclusions and other data entities via multimodal data integration as defined herein above. In specific embodiments, the combination input data may, based on suitable weighing algorithms, result in a further, more complete overall diagnostic and/or monitoring conclusion.

In a further aspect the present invention relates to the use of a nucleic acid affinity ligand and/or a peptide affinity ligand for the expression product or protein of each biomarker of a group of biomarkers, the group comprising at least Prolactin and Transferrin, and optionally also any one or more of NSE, CYFRA 21-1, CEA, and the glycolipid marker CA19-9 for detecting, diagnosing, or monitoring a cancer disease. The nucleic acid affinity ligand and peptide affinity ligand for the expression products correspond to those defined herein above. Further contemplated is the use of the biomarkers Prolactin and Transferrin for diagnosing, detecting, or monitoring a cancer disease, preferably breast cancer. Also envisaged is the use of the biomarkers Prolactin and Transferrin and any one or more of NSE, CYFRA 21-1, CEA, and the glycolipid marker CA19-9 for diagnosing, detecting, or monitoring a cancer disease, preferably breast cancer.

The following figures and example are provided for illustrative purposes. It is thus understood that the figures and example are not to be construed as limiting. The skilled person in the art will clearly be able to envisage further modifications of the principles laid out herein.

### EXAMPLE

### BREAST CANCER DETECTION PROCEDURE

Serum was generated from 5 ml blood that was centrifuged at 2000g for 10 min at 4°C and stored at -80°C. In total, 18-20 serum samples from breast cancer patients, and 6 healthy serum samples were analyzed.

The protein levels of Prolactin, Neuron Specific Enolase (NSE), Cytokeratin-19 (CYFRA 21-1), Carcinoembryonic Antigen (CEA), Transferrin and Carbohydrate antigen 19-9 (CA19-9) were determined in the serum samples by using commercially available immunoassays for each marker. All immunoassays were conducted on an Atellica IM analyzer (Siemens Healthineers Diagnostics Inc.) in accordance with the manufacturer's instructions. The operators were blinded to the blood sample results, but not to clinical characteristics.

The measurement ranges of the commercially available immunoassays were as follows:

| | |
|---|---|
| CEA (Carcinoembryonic Antigen): | 0,50-100 ng/mL |
| Prolactin: | 0,30-200 ng/mL |
| NSE (Neuron Specific Enolase): | 1,6-400 ng/mL |
| CYFRA 21-1 (Cytokeratin-19): | 0,50-100 ng/mL |
| Transferrin: | 1-440 mg/dL |
| CA19-9 (Carbohydrate antigen 19-9): | 1,2-700 U/mL |

The results indicate that the protein abundance of the described biomarkers differs in healthy and in tumor liquid biopsy serum samples.

## Claims

1. A composition for diagnosing, detecting, or monitoring a cancer disease, comprising a nucleic acid affinity ligand and/or a peptide affinity ligand for an expression product or a protein of each biomarker of a group of biomarkers, the group comprising at least Prolactin and Transferrin.

2. The composition of claim 1, additionally comprising a nucleic acid affinity ligand and/or a peptide affinity ligand for an expression product or a protein of one or more of Neuron Specific Enolase (NSE), CYFRA 21-1, Carcinoembryonic Antigen (CEA), and the glycolipid marker CA19-9.

3. The composition of claim 1 or 2, wherein said nucleic acid affinity ligand is a set of oligonucleotides specific for a Prolactin, NSE, CYFRA 21-1, CEA, or Transferrin expression product or an oligonucleotide probe specific for a Prolactin, NSE, CYFRA 21-1, CEA, or Transferrin expression product, and/or wherein the peptide affinity ligand is an antibody specific for a Prolactin, NSE, CYFRA 21-1, CEA, or Transferrin protein or the glycolipid marker CA19-9.

4. A method of diagnosing, detecting or monitoring a cancer disease in a subject, comprising at least the step of determining the level of expression of Prolactin and Transferrin, and optionally also of any one or more of NSE, CYFRA 21-1, CEA, and the glycolipid marker CA19-9 in a subject's sample, preferably comprising the additional step of determining the level of expression of Prolactin and Transferrin, and optionally also of any one or more of NSE, CYFRA 21-1, CEA, and the glycolipid marker CA19-9, in a control sample and determining the difference of the levels of expression of Prolactin and Transferrin, and optionally also of any one or more of NSE, CYFRA 21-1, CEA, and the glycolipid marker CA19-9, in the subject's sample versus the control sample.

5. A method for diagnosing, detecting or monitoring a cancer disease in a subject, comprising:
(a) determining the level of expression of Prolactin and Transferrin, and optionally also of any one or more of NSE, CYFRA 21-1, CEA, and the glycolipid marker CA19-9, in a sample of the subject;
(b) determining the level of expression of a reference marker in the subject's sample; or determining the level of an internal standard;
(c) optionally normalizing the determined expression level of Prolactin and Transferrin, and optionally also of any one or more of NSE, CYFRA 21-1, CEA, and the glycolipid marker CA19-9, to the expression level of the reference marker or internal standard; and
(d) comparing the determined expression levels of step (a) and step (b), and optionally of step (c), wherein an increased expression level of Prolactin, and optionally of any one or more of NSE, CYFRA 21-1, CEA, and the glycolipid marker CA19-9, and a decreased expression level of Transferrin in the sample of the subject compared to a control sample s indicative of a cancer disease.

6. A method for detecting, diagnosing or monitoring a cancer disease in a subject comprising at least the steps:
(a) determining the level of expression of Prolactin and Transferrin, and optionally also of any one or more of NSE, CYFRA 21-1, CEA, and the glycolipid marker CA19-9, in a sample of the subject;
(b) determining the level of expression of Prolactin and Transferrin, and optionally also of any one or more of NSE, CYFRA 21-1, CEA, and the glycolipid marker CA19-9, in a control sample;
(c) determining the difference in the expression levels of Prolactin and Transferrin, and optionally also of any one or more of NSE, CYFRA 21-1, CEA, and the glycolipid marker CA19-9, as determined in steps (a) and (b); and
(d) determining the absence or presence of a cancer disease based on the results obtained in step (c).

7. A method of any one of claims 4 to 6, wherein said method comprises the additional step of determining the expression level of one or more further biomarkers.

8. The method of claim 7, wherein said one or more further biomarker is TNFalpha, IGF-BP3, IGF1, IL8, IL10, IL2R and/or IL1b.

9. The method of any one of claims 4 to 8, wherein said sample of a subject is a body fluid sample, preferably a blood sample such as a serum or plasma sample, a urine sample, a urine sediment sample, a breath sample, a saliva sample, a semen sample, or a nipple aspiration sample.

10. The method of any one of claims 4 to 9, wherein said control sample is a sample of a healthy subject.

11. The method of any one of claims 5 and 7 to 10, wherein said reference marker is the expression product of a housekeeping gene.

12. The method of any one of claims 4 to 11, wherein the levels of expression are measured by an immunoassay such as an enzyme immunoassay (EIA), a radioimmunoassay (RUA), a fluoroimmunoassay (FIA), a chemiluminescence immunoassay (CLIA), a counting immunoassay (CIA), or an Enyzme-linked Immunosorbent Assay (ELISA), wherein said immunoassay is preferably performed on an analyzer instrument.

13. The method of any one of claims 4 to 12, additionally comprising the step of evaluation of
(i) the subject's imaging-based data, preferably derived from an MRT, medical images and/or pathology images, or mammography analysis; and/or
(ii) non-imaging-based data, such as data on age, sex, race, characteristics of cancer phenotype, histologic characteristics, histologic subtype, detectable molecular changes, preferably on the level of the genome, transcriptome, metabolome, proteome, glycome or lipidome, biomarkers and/or laboratory tests;
wherein the results of such evaluation(s) contribute to the diagnostic and/or monitoring conclusion with respect to the absence or presence of a cancer disease in the subject.

14. Use of a nucleic acid affinity ligand and/or a peptide affinity ligand for an expression product or protein of each biomarker of a group of biomarkers, the group comprising at least Prolactin and Transferrin, and optionally also any one or more of NSE, CYFRA 21-1, CEA, and the glycolipid marker CA19-9, for detecting, diagnosing, or monitoring a cancer disease.

15. The composition of claim 1, 2 or 3, the method of any one of claims 4 to 13, or the use of claim 14, wherein said cancer disease is breast cancer, preferably an early form of breast cancer.
